# DEMANDE DE BREVET EUROPEEN

(11) **EP 2 187 112 A1**
(43) Date de publication de la demande: **19.05.2010**
(21) Numéro de dépôt: 09306076.2
(22) Date de dépôt: 10.11.2009
(51) Int. Cl.: F21S 8/00, F21K 99/00, A61N 5/06, G01R 31/26, H05B 33/08, F21Y 101/02

(54) **Procédé de sélection de LEDs pour produire d'un spectre lumineux prédéterminé panneau de LEDs correspondant**

(30) Priorité: 14.11.2008 FR 0806332
(71) Demandeur: Société J.F. Cesbron Holding, 49124 Saint-Barthélémy-d'Anjou (FR); Centre National de la Recherche Scientifique, 75794 Paris Cedex 16 (FR); Versatel Technologies, 49070 Beaucouze (FR)
(72) Inventeur: Verdier, Bruno, 77000, MELUN (FR); Jouanneau, Isabelle, 77140, ST PIERRE LES NEMOURS (FR); Rabin, Christian, 72200, LA FLECHE (FR); Simonnet, Benoît, 44300, NANTES (FR); Verchan, Michel, 49000, ANGERS (FR)
(74) Mandataire: Laget, Jean-Loup

(57) **Abrégé**

L'invention concerne un procédé de reproduction d'un spectre lumineux dit de référence (SR), à l'aide d'au moins une carte (1) support de LEDs. Ledit procédé comporte au moins les étapes suivantes :
a) on allume un nombre donné de LEDs d'une même couleur, et on enregistre le spectre, dit élémentaire, obtenu avec lesdites LEDs allumées,
b) on répète plusieurs fois l'étape a) avec au moins des LEDs d'une couleur différente à chaque fois,
c) on simule des spectres de prédiction en affectant à chaque spectre élémentaire un coefficient et en sommant lesdits spectres élémentaires affectés de leur coefficient,
d) on identifie le spectre de prédiction simulé qui s'approche le plus du spectre de référence à reproduire,
e) on détermine les proportions relatives de LEDs de chaque couleur correspondant au spectre de prédiction identifié,
f) on dispose lesdites LEDs sur la carte.

## Description

La présente invention concerne de manière générale la reproduction de spectres de lumière correspondant à une courbe de flux de rayonnement en fonction de la longueur d'onde, tel que le spectre du soleil.

Afin d'observer l'évolution d'un écosystème dans un environnement particulier, il est souhaitable de pouvoir reconstituer les données environnementales auxquelles ledit écosystème est soumis, en particulier le spectre de lumière, tel que le spectre solaire, que reçoit ledit écosystème.

Jusqu'ici le spectre solaire était simulé à l'aide de tubes de néon. Une telle solution n'est pas satisfaisante car le spectre d'un néon est composé de plusieurs pics ponctuels et forme un spectre très éloigné du spectre solaire. En outre, un éclairage par néons ne permet pas de reproduire une pluralité de spectres différents.

En particulier, lorsque l'écosystème est formé de plantes, celles-ci nécessitent une puissance lumineuse minimale de 20 W.m⁻² pour se développer par photosynthèse. Les néons couramment utilisés ne permettent pas d'atteindre cette puissance.

On connaît des documents US-2006/018118, DE-10 2005 059362 et WO 2006/119750 des cartes équipées d'une pluralité de LEDs de différentes couleurs. Cependant, dans ces documents, le choix des couleurs de LEDs et le choix du nombre de LEDs d'une couleur donnée s'effectue de manière empirique. Le plus souvent lorsque l'on souhaite générer un spectre de lumière donné, la distribution des LEDs n'est pas optimisée pour ledit spectre et l'opérateur doit jouer sur les intensités des LEDs pour tenter d'obtenir un spectre proche du spectre souhaité. En outre, le fait de jouer simplement sur les intensités des LEDs ne suffit généralement pas pour obtenir de manière fiable et efficace le spectre souhaité. En effet, le pilotage de l'intensité des LEDs ne permet de faire varier le spectre généré par les différentes LEDs que dans un domaine de spectre limité par la distribution initiale des LEDs.

La présente invention a pour but de permettre de déterminer de manière rapide, fiable et répétable les quantités relatives de LEDs de chaque couleur à positionner sur la carte pour obtenir le spectre souhaité.

Un autre but de l'invention est de permettre la reproduction de manière aisée et rapide de différents spectres de lumière.

A cet effet, l'invention concerne un procédé de reproduction d'un spectre lumineux dit de référence, correspondant à une courbe de flux de rayonnement en fonction de la longueur d'onde, tel que le spectre du soleil à un moment donné et à un endroit donné,
ledit spectre lumineux étant reproduit à l'aide d'au moins une carte support de diodes électroluminescentes, appelées LEDs, ledit procédé comportant au moins les étapes suivantes :
a) on allume un nombre donné de LEDs d'une même couleur et on enregistre le spectre, dit élémentaire, obtenu avec lesdites LEDs allumées,
b) on répète plusieurs fois l'étape a) avec au moins des LEDs d'une couleur différente à chaque fois,
c) on simule des spectres dits de prédiction, chaque spectre de prédiction étant obtenu en affectant au flux de rayonnement de chaque spectre élémentaire enregistré un coefficient et en sommant lesdits spectres élémentaires affectés de leur coefficient,
d) on identifie parmi les spectres de prédiction simulés, celui qui s'approche le plus du spectre de référence à reproduire,
e) on détermine les proportions relatives de LEDs de chaque couleur à positionner sur ladite au moins une carte à partir au moins du coefficient affecté à, et si nécessaire du nombre de LEDs de, chaque spectre élémentaire du spectre de prédiction identifié comme s'approchant le plus du spectre de référence à reproduire,
f) on dispose sur ladite au moins une carte les LEDs des différentes couleurs selon les proportions relatives déterminées.

Autrement dit, pour différentes couleurs, on enregistre le spectre dit élémentaire que l'on obtient avec un nombre donné de LEDs de même couleur, ce qui permet de réaliser une base de données de spectres élémentaires. Ces spectres élémentaires sont ensuite affectés de coefficients et additionnés suivant leurs longueurs d'onde pour composer des spectres de prédiction. Le spectre de prédiction qui s'approche le plus du spectre de référence est identifié et le jeu de coefficients qui a permis d'obtenir un tel spectre de prédiction est utilisé pour déterminer la quantité ou nombre de LEDs de chaque couleur à distribuer sur la carte.

La mémorisation de spectres élémentaires permet de former une banque ou base de données que l'on peut compléter continuellement et plus cette base de données est importante, plus le nombre de spectres que l'on peut simuler avec précision est important. Ainsi plus la base de données est importante plus la variété de spectres que l'on peut produire de manière fiable est importante. Il suffit en effet comme rappelé ci-dessus de convertir les coefficients du spectre simulé qui s'approche le plus du spectre souhaité en des nombres de LEDs pour les couleurs associées auxdits coefficients. Le procédé selon l'invention permet ainsi de reproduire de manière fiable et rapide une grande variété de spectres de lumière.

Un tel procédé permet de reconstituer aisément, de manière fiable et rapide, un spectre de référence donné, à l'aide de la base de données de spectres élémentaires. Les spectres simulés sont générés de manière fiable et rapide en combinant entre eux lesdits spectres élémentaires affectés de coefficients. En particulier, la combinaison des spectres élémentaires tient compte des chevauchements de plages de longueur d'onde en sommant les irradiances des différents spectres pour chaque longueur d'onde comme expliqué ci-après.

Le spectre de prédiction simulé le plus proche du spectre souhaité peut alors être aisément obtenu en positionnant les quantités relatives de LEDs de couleurs correspondantes sur la carte. Ce procédé peut être mis en oeuvre pour reproduire différents spectres de lumière artificiels ou naturels, en particulier le spectre solaire.

Dans l'état de la technique, le choix empirique des LEDs en nombre et en couleur ne permet pas d'identifier de manière fiable la combinaison de LEDs optimale et en particulier ne permet pas de prendre en compte les conséquences sur le spectre global généré par les LEDs des chevauchements des plages de longueur d'onde des différentes couleurs de LEDs.

Contrairement aux procédés de reproduction de spectre lumineux de l'état de la technique, le procédé selon l'invention permet, dès réalisation de la carte de LEDs, d'optimiser avec une méthode fiable rapide et efficace le choix des couleurs et des nombres de LEDs sur la carte en vue d'obtenir le spectre souhaité. Ainsi, avec le procédé selon l'invention, pour obtenir le spectre souhaité, il n'est pas nécessaire que l'opérateur pilote les intensités des LEDs positionnées sur la carte, même s'il en a la possibilité pour modifier ledit spectre.

Selon une caractéristique avantageuse de l'invention, la carte utilisée étant une carte présentant des emplacements de réception de LEDs répartis de manière régulière, en ligne et en colonne, ou en triangles équilatéraux, on dispose les LEDs d'une même couleur de telle sorte que lesdites LEDs ou les groupes de LEDs de ladite couleur soient sensiblement équidistants les un(e)s des autres.

Autrement dit, on dispose les LEDs d'une même couleur avec un écartement sensiblement constant des LEDs ou groupes de LEDs entre elles (eux) en ligne et/ou en colonne et/ou en diagonale. Un montage à équidistance des LEDs ou de groupes LEDs permet d'obtenir une bonne homogénéité du spectre émis par chaque zone d'une carte.

Selon une caractéristique avantageuse de l'invention, on monte les LEDs, une à une ou par modules formés de plusieurs LEDs, de manière amovible sur la carte, de préférence par encliquetage.

Le montage de manière amovible des LEDs permet d'agencer à volonté les LEDs sur une même carte en fonction du spectre à réaliser. Une telle conception permet également de remplacer des LEDs défectueuses sans avoir à remplacer toute la carte. En outre, le montage amovible des LEDs permet de tester différentes distributions de LEDs de différentes couleurs.

Selon une caractéristique avantageuse de l'invention, on intercale sur ladite au moins une carte support les LEDs ou groupes de LEDs d'une même couleur entre des LEDs ou groupes de LEDs d'une ou de plusieurs autres couleurs.

Une telle configuration des LEDs favorise la répartition des LEDs de chaque couleur sur l'ensemble de la carte et évite la formation de zones de concentration de certaines couleurs.

Selon une caractéristique avantageuse de l'invention, on dispose sur la carte, en plus des LEDs de couleur dont les proportions ont été déterminées à la suite des étapes c) a à f), des LEDs supplémentaires appelées LEDs de réserve.

Selon une caractéristique avantageuse de l'invention, pour générer un nouveau spectre à partir des LEDs présentes sur la carte, on simule des spectres de prédiction à partir des LEDs présentes et notamment des LEDs de réserve pour identifier la combinaison de LEDs permettant d'obtenir un spectre simulé le plus proche du nouveau spectre souhaité.

Selon une caractéristique avantageuse de l'invention, on mesure le spectre émis correspondant à la disposition des LEDs sur la carte réalisée à l'étape f), et on modifie la puissance émise par au moins une partie des LEDs, et/ou on pilote au moins une partie des LEDs de réserve lorsqu'elles sont présentes, pour réduire l'écart entre le spectre de référence et le spectre obtenu avec les LEDs disposées sur la carte.

La modification de la puissance lumineuse émise par certaines LEDs permet d'affiner le spectre émis par la carte de LEDs de manière à s'approcher encore mieux du spectre de référence à reproduire.

L'invention concerne également une carte obtenue selon le procédé décrit ci-dessus. Préférentiellement, la carte comporte des emplacements de réception de LEDS qui sont conçus pour un montage amovible des LEDs ou de modules formés de plusieurs LEDs, de préférence par encliquetage.

Avantageusement, ladite carte est équipée de moyens de pilotage de l'alimentation électrique de chaque LED indépendamment des autres LEDs de la carte ou de chaque module de LEDs indépendamment des autres modules de LEDs de la carte.

Une telle conception de la ou de chaque carte offre une grande liberté de pilotage des LEDs et ainsi de nombreuses possibilités de réglage de la qualité du spectre émis par la distribution de LEDs sur la ou chaque carte obtenue par le procédé selon l'invention.

L'invention concerne également un panneau formé de cartes de LEDs telles que décrites ci-dessus, disposées sensiblement côte à côte pour former une surface d'éclairage.

Selon une caractéristique avantageuse du panneau selon l'invention, la répartition des LEDs est sensiblement identique d'une carte à une autre. Un tel panneau autorise la reproduction d'un spectre de lumière donné sur une surface importante, de manière homogène sur toute la surface.

Lesdites cartes, en particulier lorsqu'elles sont de format rectangulaire ou carré, peuvent être juxtaposées en lignes et en colonnes. En variante, lesdites cartes peuvent être de forme triangulaire, de préférence équilatérale. Dans ce cas, le panneau est formé de rangées de cartes triangulaires agencées tête-bêche pour minimiser l'espace laissé libre entre deux cartes.

Selon une caractéristique avantageuse de l'invention, la distribution de LEDs sur une partie des cartes correspond à un spectre de référence donné et la distribution de LEDs sur une autre partie des cartes correspond à un autre spectre de référence donné.

Selon une caractéristique avantageuse de l'invention, le panneau comporte des moyens de pilotage conçus pour permettre un pilotage des LEDs de chaque carte indépendamment des LEDs des autres cartes et, de préférence, conçus pour permettre un pilotage de chaque LED d'une carte indépendamment des autres LEDs de ladite carte. Avantageusement, lesdits moyens de pilotage comportent des moyens de réplication des consignes d'alimentation des LEDs d'une carte aux autres cartes de manière à obtenir un spectre le plus uniforme possible d'une carte à une autre.

Selon une caractéristique avantageuse de l'invention, ledit panneau est monté mobile à translation selon une direction normale au plan dudit panneau, de manière à permettre un réglage de la distance entre l'ensemble des LEDs dudit panneau et le ou les éléments destinés à être éclairés par ledit panneau.

L'invention concerne également un procédé de pilotage d'une carte telle que décrite ci-dessus. Ledit procédé de pilotage s'applique également à un panneau tel que décrit ci-dessus. Selon ce procédé de pilotage, on éteint ou on fait varier l'intensité d'alimentation d'au moins une partie des LEDs de manière à modifier la qualité du spectre.

L'invention sera bien comprise à la lecture de la description suivante d'exemples de réalisation, en référence aux dessins annexés dans lesquels :
- la figure 1 est une vue de face d'un panneau de LEDs selon un mode de réalisation de l'invention pour lequel chaque carte de LEDs est rectangulaire;
- la figure 1A est une vue de face d'un panneau de LEDs selon un autre mode de réalisation de l'invention pour lequel chaque carte de LEDs est triangulaire ;
- la figure 2 est un graphique montrant la courbe du spectre de référence et celle du spectre de prédiction le plus proche dudit spectre de référence, obtenu par le procédé selon l'invention ;
- les figures 3A à 3E illustrent chacune, pour une couleur donnée, le spectre élémentaire enregistré pour un nombre donné de LEDs d'une même couleur, l'autre courbe correspondant audit spectre élémentaire affecté d'un coefficient ;
- la figure 4 est une vue d'une carte de LEDs selon un mode de réalisation particulier de l'invention pour lequel les LEDs sont réparties par groupe de quatre et de six sur ladite carte ;
- les figures 5A, 5B, 6A, 6B, et 7A, 7B illustrent différents agencements de LEDs, lesdites LEDs d'une même couleur étant réparties de manière équidistante les unes par rapport aux autres ou de manière à former des groupes de LEDs équidistants les uns par rapport aux autres,
- la figure 8 est une vue des moyens de déplacement du panneau de LEDs par rapport à l'écosystème à éclairer.

Par LEDs d'une même couleur, on entend des LEDs d'une même longueur d'onde ou d'une même composition de longueurs d'onde.

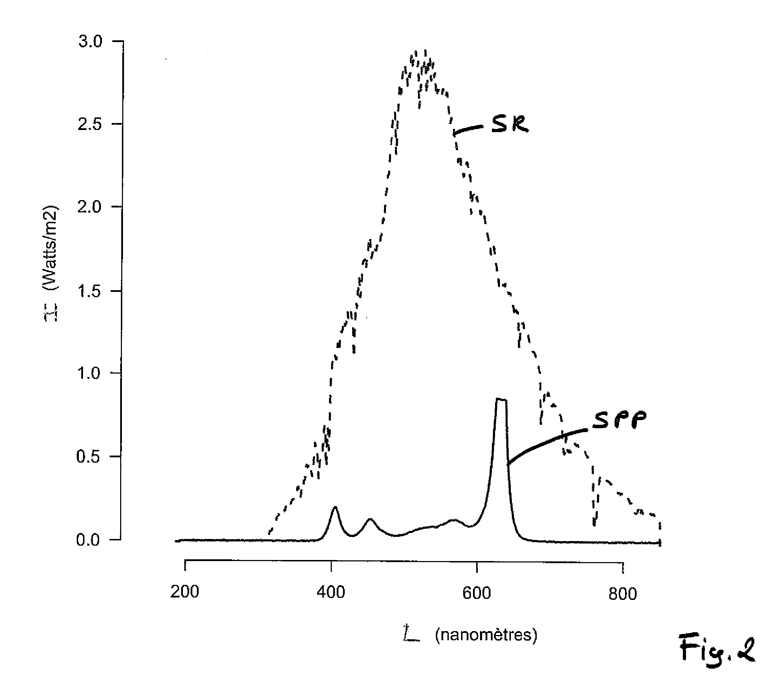

Comme rappelé ci-dessus et en référence aux figures, l'invention concerne un procédé de reproduction d'un spectre lumineux dit de référence, correspondant à une courbe de flux de rayonnement I en fonction de la longueur d'onde L. Dans l'exemple illustré aux figures, en particulier à la figure 2, le spectre de référence SR à reproduire est le spectre du soleil à un moment donné et à un endroit donné, par exemple à Paris à 11h.

Comme détaillé ci-après, ledit spectre lumineux est reproduit à l'aide de cartes 1 support de LEDs 21, 22, 23, 24, 25. Lesdites cartes sont agencées les unes à côté des autres pour former un panneau 100 de LEDs. On peut prévoir de laisser un espace entre chacune des cartes de manière à réduire les risques d'interférence lumineuse au niveau des spectres émis par des cartes voisines. Ledit espace peut également servir à créer une zone libre pour la ventilation entre lesdites cartes.

Le procédé qui permet de reproduire le spectre de référence à l'aide d'une carte de LEDs comporte au moins les étapes suivantes :
a) on allume un nombre donné de LEDs 21 d'une même couleur, lesdites LEDs allumées étant disposées sur une carte de test, et on enregistre le spectre SE1, dit élémentaire, obtenu avec lesdites LEDs 21 allumées, dans une mémoire d'un système informatique,
b) on répète plusieurs fois l'étape a) avec au moins des LEDs 21 ; 22 ; 23 ; 24; 25 d'une couleur différente à chaque fois,
c) on simule à l'aide d'un système informatique des spectres dits de prédiction, chaque spectre de prédiction étant obtenu en affectant au flux de rayonnement de chaque spectre élémentaire SE1, SE2, SE3, SE4, SE5 enregistré un coefficient et en sommant lesdits spectres élémentaires affectés de leur coefficient,
d) on identifie parmi les spectres de prédiction simulés, celui SPP qui s'approche le plus du spectre de référence SR à reproduire,
e) on détermine les proportions relatives de LEDs, en nombre par couleur, à positionner sur la carte à partir au moins du coefficient affecté à chaque spectre élémentaire du spectre de prédiction SPP identifié comme s'approchant le plus du spectre de référence SR à reproduire, et si nécessaire à partir du nombre de LEDs pour lequel a été enregistré chaque spectre élémentaire ;
f) on dispose sur la carte les LEDs des différentes couleurs selon les proportions relatives déterminées.

Ledit procédé est mis en oeuvre à l'aide d'un système informatique comportant une mémoire de stockage de données pour mémoriser notamment lesdits spectres élémentaires et des moyens de calcul, tels qu'un microprocesseur, pour simuler les spectres de prédiction et identifier le spectre simulé le plus proche du spectre souhaité. Ledit système informatique forme une unité de détermination des LEDs à utiliser pour obtenir un spectre souhaité. Le spectre souhaité est également mémorisé dans la mémoire de manière à permettre aux moyens de calcul de comparer les spectres simulés au spectre souhaité. Ledit microprocesseur est programmé avec des instructions pour la mise en oeuvre des étapes a) à d) et éventuellement de l'étape e).

Une fois positionnées sur la carte, les LEDs permettant d'obtenir le spectre souhaité, ladite unité de détermination des LEDs peut de nouveau être utilisée en vue de générer un nouveau spectre à partir des LEDs déjà présentes sur la carte et éventuellement de LEDs supplémentaires, dites LEDs de réserve, comme expliqué ci-après. En effet, pour un nouveau spectre souhaité, ladite unité est configurée pour déterminer le nombre de LEDs de chaque couleur à allumer pour obtenir ledit nouveau spectre souhaité.

Eventuellement, ladite unité peut être également configurée pour piloter l'intensité desdites LEDs de manière à affiner le spectre généré par les LEDs afin d'obtenir le nouveau spectre souhaité.

L'utilisation de LEDs de différentes couleurs réparties sur une carte permet de bénéficier d'une large gamme de longueurs d'onde pour chacune desquelles on peut modifier le flux de rayonnement, ce qui permet de faire varier au cours du temps la qualité ou la nature du spectre de lumière auquel est soumis l'écosystème. Un spectre de référence donné peut ainsi être reproduit de manière bien plus précise qu'avec des néons. Chaque carte obtenue avec un tel procédé forme un système d'éclairage émettant un spectre dont on peut faire varier au cours du temps la qualité. En outre, l'utilisation de diodes permet d'atteindre une puissance minimale de 20 W.m⁻² autorisant le développement de plantes par photosynthèse.

Les étapes de ce procédé sont détaillées ci-dessous.

### Etapes a) et b)

Le flux de rayonnement enregistré en fonction de la longueur d'onde correspond dans l'exemple illustré aux figures à l'irradiance. L'irradiance se définit comme l'énergie reçue par le système de mesure ou l'écosystème en watts par mètre carré. Le système de mesure utilisé est un spectromètre calibré en irradiance relié au système informatique formant l'unité de détermination des LEDs à utiliser. Ainsi, chaque spectre lumineux est mesuré par un instrument permettant de mesurer l'irradiance en fonction de la longueur d'onde. Chaque spectre lumineux est alors mesuré puis enregistré dans la mémoire du système informatique pour constituer une base de données qui peut être enrichie à tout moment.

Dans l'exemple illustré ci-dessous, les LEDs de couleurs pour lesquelles un spectre élémentaire a été enregistré sont au moins formées des LEDs 21 ; 22 ; 23 ; 24; 25 suivantes :
- LEDs ultraviolets (UV), référencées 21 aux figures;
- LEDs rouge "foncé" émettant autour de 660 nm, référencées 22;
- LEDs rouge " clair" émettant autour de 625 nm, référencées 23;
- LED infrarouges, référencées 24;
- LEDs blanches émettant sur la plage 453-550nm, référencées 25.

Pour chacune de ces couleurs, on a enregistré le spectre lumineux émis par une disposition de cent LEDs de ladite couleur sur une carte comprenant 100 emplacements. Préférentiellement, les cent LEDs sont réparties de manière matricielle c'est-à-dire en dix colonnes et en dix lignes.

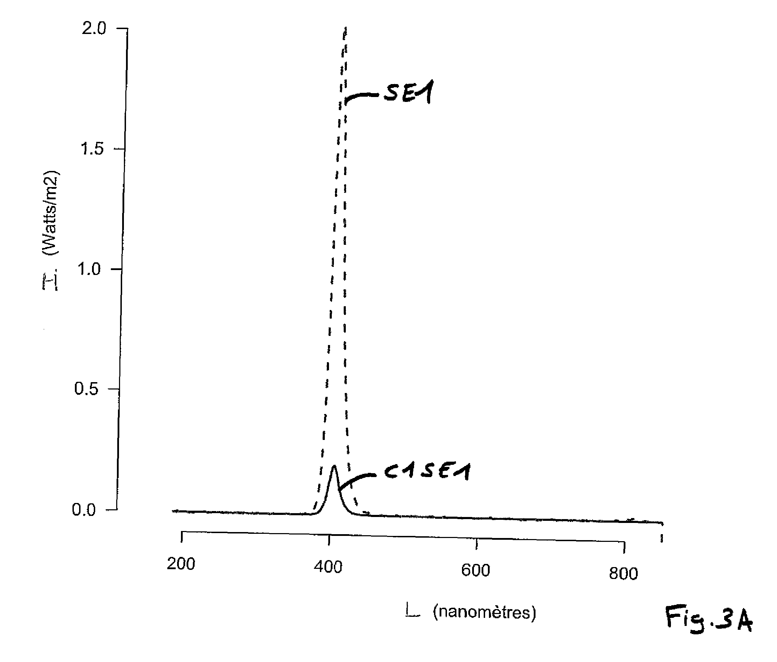

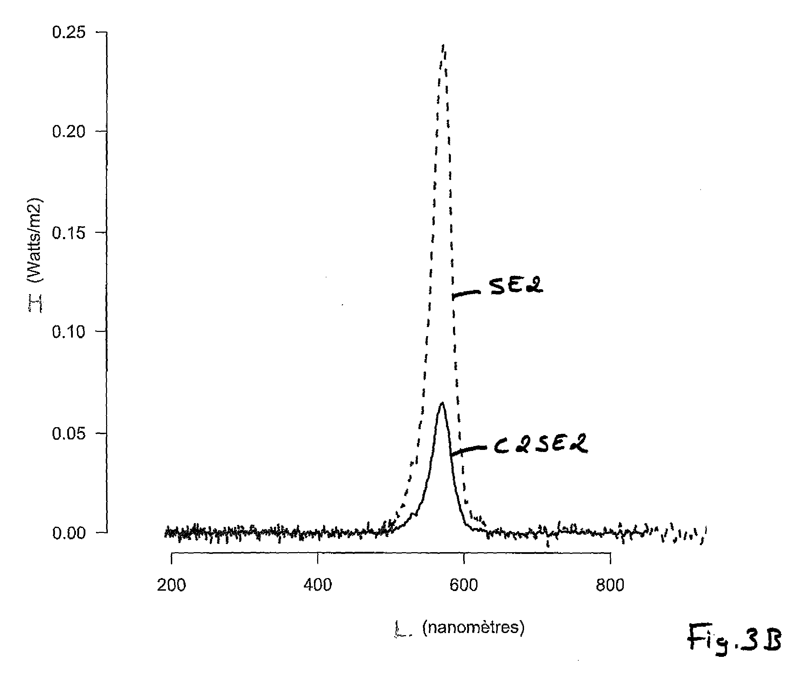

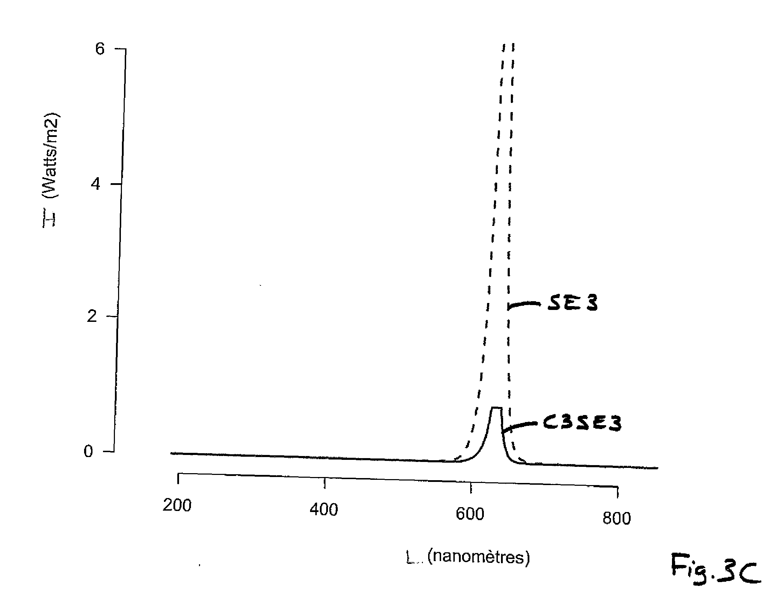

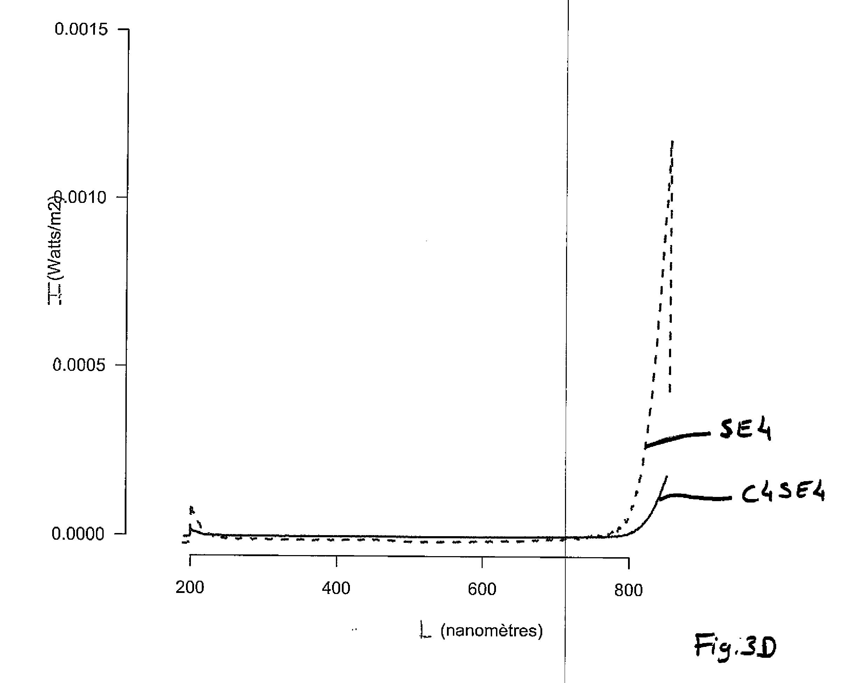

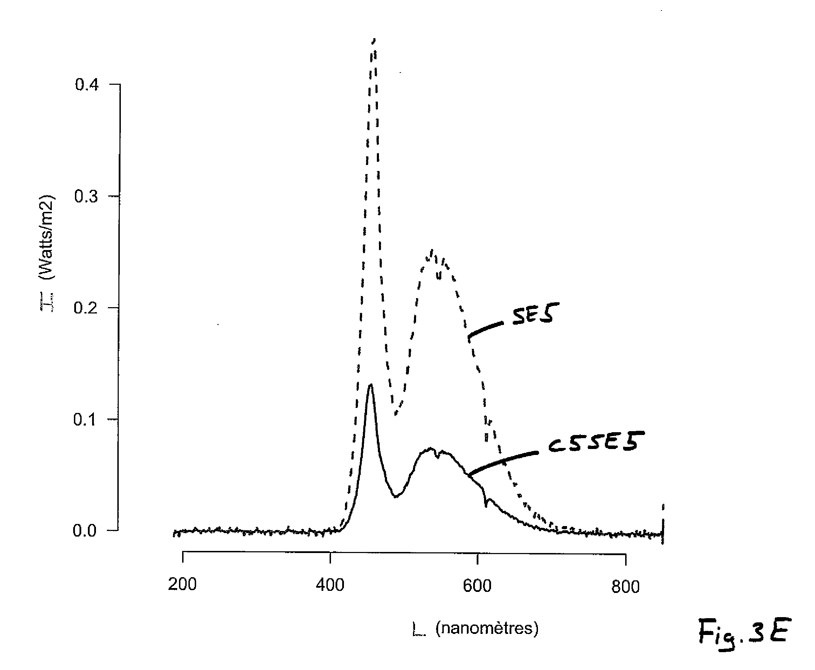

On a ainsi enregistré pour les couleurs rappelées ci-dessus les spectres élémentaires correspondants illustrés aux figures 3A à 3E par les courbes SE1, SE2, SE3, SE4, SE5. Lesdites courbes SE1, SE2, SE3, SE4, SE5 correspondent respectivement aux spectres (pour cent LEDs) des couleurs, ultraviolet (UV), rouge "foncé", rouge "clair", infrarouges et blanche. A la figure 3D, le spectre infrarouge représenté est interrompu du fait que le spectromètre utilisé ne réalisait pas d'acquisition au-delà de 850 nanomètres.

Les spectres élémentaires des différentes couleurs ont été enregistrés pour une distance donnée entre la carte et l'instrument de mesure du spectre. Ladite distance donnée est de préférence choisie à partir d'enregistrements de spectres élémentaires précédemment réalisés pour différentes distances. La distance entre le panneau de diodes et le système à éclairer est choisie de manière à obtenir une répartition de couleurs émises suffisamment homogène tout en conservant une puissance d'émission suffisante.

En outre, lors d'essais préparatoires, pour chaque couleur, d'autres spectres élémentaires ont également été enregistrés avec des nombres de LEDs différents, en faisant également varier la distance entre la carte de LEDs et l'instrument de mesure du spectre. Une telle mesure du spectre émis par un ensemble de LEDs d'une même couleur, en fonction de la distance et pour différents nombres de LEDs permet de vérifier que les effets de bord et que les cônes d'émission des LEDs sont suffisamment faibles pour ne pas créer d'interférence entre les LEDs. On observe en effet que la puissance émise par les LEDs portées par la carte varie sensiblement de la même manière avec la distance pour des nombres de LEDs différents. L'énergie reçue par un système est inversement proportionnelle au carré de sa distance à la source lumineuse.

Ainsi, les étapes a) et b) permettent d'obtenir une base de données de spectres élémentaires pour différentes LEDs. Les différentes LEDs dont le spectre élémentaire est enregistré comprennent au moins des LEDs de différentes couleurs conformément aux étapes a) et b), et il est également possible d'enregistrer des spectres élémentaires de LEDs présentant des propriétés optiques différentes, par exemple deux types de LEDs qui, bien qu'elles émettent sensiblement la même couleur, présentent des puissances lumineuses ou des cônes d'émission différents d'un type de LED à l'autre.

### Etape c)

Pour chaque spectre élémentaire SE1, SE2, SE3, SE4, SE5 de couleur enregistré précédemment, on applique pour chaque longueur d'onde composant ledit spectre élémentaire enregistré un coefficient à la valeur d'irradiance associée à ladite longueur d'onde. On obtient ainsi les spectres dits révisés C1SE1, C2SE2, C3SE3, C4SE4, C5SE5 illustrés aux figures 3A à 3E. Une fois cette opération réalisée pour chaque spectre élémentaire enregistré, les spectres élémentaires ainsi révisés sont superposés et pour chaque longueur d'onde commune à deux spectres élémentaires, on additionne les irradiances correspondantes. On obtient ainsi un spectre de prédiction correspondant à un mélange donné de LEDs de couleurs. Cette opération de génération d'un spectre de prédiction est réalisée à l'aide d'un modèle mathématique programmé sous forme d'instructions de calcul. Ledit modèle est une boucle qui change les coefficients affectés aux spectres élémentaires enregistrés automatiquement et, de préférence, de manière à ce que la somme des coefficients soit toujours égale à 1, de manière à obtenir une pluralité de spectres de prédiction.

Lorsque l'on ne souhaite pas tenir compte de certains spectres élémentaires enregistrés, il est possible de leur affecter un coefficient nul.

Comme détaillé ci-après, on obtient ainsi de manière fiable un grand nombre de spectres simulés et il suffit d'identifier le spectre simulé le plus proche du spectre souhaité pour récupérer les coefficients qui ont permis d'obtenir ce spectre et les convertir chacun en un nombre de LEDs d'une couleur donnée à positionner sur la carte comme expliqué ci-après.

### Etape d)

Chaque spectre de prédiction généré correspond au spectre théorique d'un mélange de LEDs de différentes couleurs selon des proportions relatives qui correspondent, pour chaque couleur, au coefficient affecté au spectre élémentaire de ladite couleur.

Le spectre le plus proche peut être défini comme étant le spectre de prédiction obtenu pour lequel, d'une part, le ratio des couleurs (c'est-à-dire les quantités relatives de longueur d'onde émises) est le plus proche, et, d'autre part, l'irradiance est la plus proche. On peut également prévoir de définir le spectre le plus proche comme étant celui dont la forme est la plus proche de celle du spectre de référence, et dont l'irradiance totale est la plus proche de celle du spectre de référence.

Dans l'exemple illustré aux figures, le spectre de prédiction SPP le plus proche correspond à un spectre obtenu avec le jeu de coefficients suivant :
0, 10 pour le spectre de LEDs UV,
0, 266 pour le spectre de LEDs rouge foncé,
0, 133 pour le spectre de LEDs rouge clair, et
0, 166 pour le spectre de LED infrarouge,
0, 33 pour le spectre de LEDs blanches.

### Etape e)

Comme rappelé ci-dessus, on détermine les proportions relatives de LEDs de chaque couleur à positionner sur le panneau à partir au moins du jeu de coefficients qui a permis de simuler le spectre de prédiction identifié comme s'approchant le plus du spectre de référence à reproduire.

Avantageusement la somme des coefficients affectés aux différents spectres élémentaires composant un spectre de prédiction est sensiblement égale à 1. Les enregistrements de spectres élémentaires ont été réalisés à chaque fois pour un même nombre de LED, ici égal à 100. Il en résulte que les coefficients du jeu de coefficient associé au spectre de prédiction identifié comme le plus proche, fournissent directement le pourcentage de LEDs de chaque couleur à répartir sur la carte.

Dans l'hypothèse où les nombres de LEDs utilisées pour les différents enregistrements de spectres élémentaires ne seraient pas les mêmes, il conviendrait de tenir compte, pour le calcul des proportions de LEDs des différentes couleurs à répartir sur la carte, des nombres de LEDs pour lesquels ont été enregistrés les spectres élémentaires. Dans ce cas, on calcule pour chaque couleur un nombre dit rectifié obtenu par multiplication du nombre de LEDs utilisées pour l'enregistrement du spectre élémentaire de ladite couleur avec le coefficient correspondant, puis on effectue le rapport du nombre rectifié correspondant à ladite couleur avec la somme des nombres rectifiés des autres couleurs, ce qui donne le pourcentage de LEDs de ladite couleur à positionner sur la carte. Par exemple, si on a fait une mesure avec 48 LEDs, on multiplie l'irradiance du spectre obtenu par 100/48 pour le ramener à un spectre de 100 LEDs.

Pour une carte de 180 emplacements, les nombres de LEDs des différentes couleurs correspondant aux proportions relatives de LEDs obtenues ci-dessus sont les suivantes :
18 LEDs UV,
48 LEDs rouges "foncé",
24 LEDs rouges "clair", et
30 LEDs infrarouges,
60 LEDs blanches.

Ainsi, le procédé selon l'invention, qui permet d'optimiser la configuration des LEDs en couleurs et en nombres, est très fiable et très efficace du fait de l'utilisation d'une importante base de données de spectres élémentaires. On obtient de manière fiable, rapide et répétable une répartition optimale de LEDs en nombres et en couleurs à positionner sur la carte comme expliqué ci-après pour obtenir, dès le départ, un spectre très proche du spectre souhaité.

### Etape f)

Préférentiellement, les emplacements ménagés dans chaque carte pour la réception des LEDs sont conçus pour un montage amovible des LEDs, par exemple par encliquetage. On monte alors les LEDs sur une face de la carte tandis que les composants et les connecteurs du système électronique et/ou informatique (voir ci-après) sont situés sur la face opposée.

Avantageusement, on dispose sur la carte les LEDs des différentes couleurs selon les proportions relatives déterminées en essayant d'intercaler les LEDs ou groupes de LEDs d'une même couleur entre des LEDs ou groupes de LEDs d'une ou de plusieurs autres couleurs. Les LEDs d'une même couleur sont avantageusement réparties sensiblement sur toute la surface de la carte de manière à ne pas créer de concentration d'une couleur dans une zone particulière de la carte. Autrement dit, les LEDs de chaque couleur sont réparties de la manière la plus homogène possible sur l'ensemble de la carte afin que chaque zone de l'écosystème éclairé par le panneau de LEDs reçoive sensiblement la même qualité et la même quantité de lumière.

On peut prévoir qu'une partie des emplacements de LEDs de chaque carte soit réservée à des LEDs dites de réserve, autres que celles déterminées à la suite dudit procédé décrit ci-dessus. Les LEDs de réserve sont formées de LEDs de différentes couleurs ou de groupe de LEDs de différentes couleurs et sont réparties de manière la plus homogène possible sur la carte. On peut par exemple prévoir de conserver 10 % des emplacements de LEDs pour lesdites LEDs de réserve. Ainsi, pour une carte de 200 emplacements, 180 emplacements peuvent être utilisés pour positionner les LEDs déterminées comme-ci-dessus.

Les LEDs de réserve peuvent être utilisées notamment pour modifier le spectre dans une certaine plage ou si besoin pour affiner le spectre souhaité. Ainsi, suivant les besoins découlant du spectre souhaité, il est possible d'allumer au moins une partie des LEDs de réserve, lorsque celles-ci sont présentes.

Les caractéristiques des LEDs de réserve, notamment leur couleur et leur position, sont bien entendu mémorisées par les moyens de pilotage pour permettre leur pilotage.

Pour obtenir un nouveau spectre lumineux modifié par rapport au spectre initial, on peut relancer les étapes c) à e), en particulier en simulant des spectres de prédiction à partir de combinaison des spectres élémentaires correspondant aux LEDs positionnées sur la carte et des spectres élémentaires des LEDs de réserve.

La combinaison de spectres élémentaires des LEDs présentes sur la carte qui permet de s'approcher le mieux du nouveau spectre souhaité est identifiée puis les LEDs correspondantes sur la carte sont allumées. Éventuellement on ajuste le spectre global réel fourni par lesdites LEDs sur la carte en pilotant leur intensité.

On peut également prévoir de mesurer le spectre résultant de la distribution des LEDs de la carte, et de modifier la puissance d'émission d'au moins une partie des LEDs pour réduire l'écart entre le spectre de référence et le spectre obtenu avec les LEDs. La modification de puissance émise est réalisée en modifiant l'intensité d'alimentation de la ou des LEDs correspondantes et/ou en pilotant les LEDs de réserve lorsqu'elles sont présentes. Eventuellement, on peut prévoir de remplacer certaines LEDs sous-dimensionnées par des LEDs de plus grande capacité permettant d'atteindre la puissance d'émission souhaitée.

Préférentiellement, après avoir obtenu une distribution des différentes LEDs sur une carte considérée comme satisfaisante au regard du spectre de référence à reproduire, on procède à la même distribution de LEDs sur chacune des autres cartes. On obtient ainsi un panneau de LEDs dont la répartition des LEDs est sensiblement identique d'une carte à une autre.

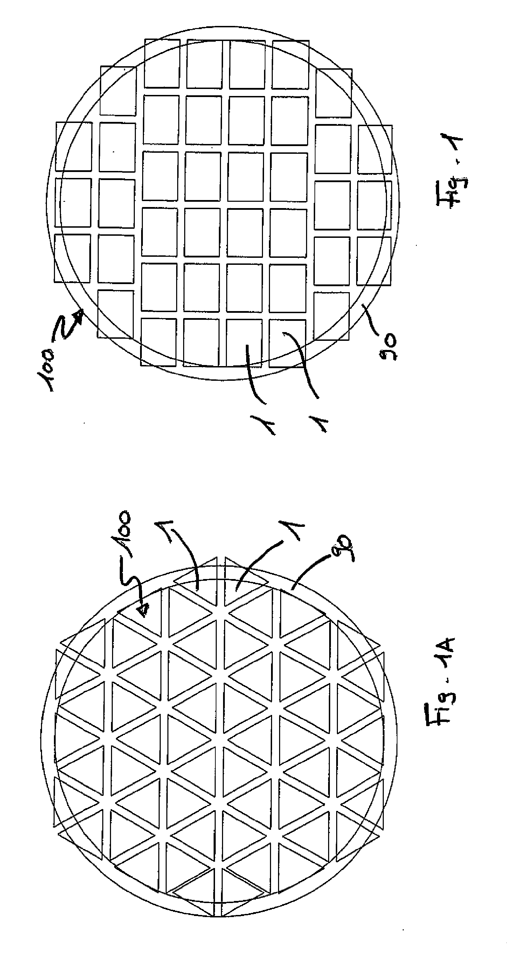

Dans l'exemple illustré à la figure 1, lesdites cartes ont un format rectangulaire et sont juxtaposées en rangées, de préférence en lignes et en colonnes.

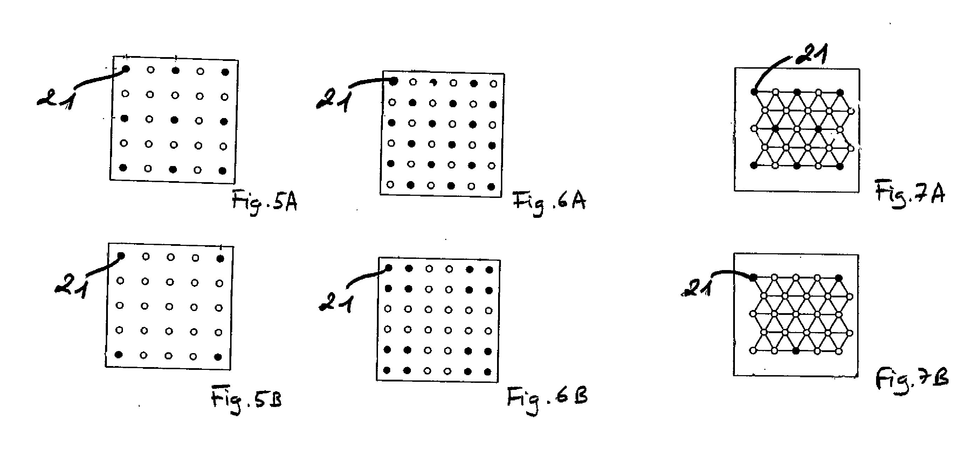

Selon un mode de réalisation particulier de l'invention, la carte utilisée est une carte dont les emplacements de réception de LEDs sont répartis de manière régulière, en ligne et en colonne (figures 5A, 5B, 6A, 6B et 8), ou en triangles équilatéraux (figure 7A, 7B). On dispose alors les LEDs d'une même couleur de telle sorte que lesdites LEDs ou les groupes de LEDs de ladite couleur soient sensiblement équidistants les unes des autres.

La figure 5A illustre un exemple de répartition à équidistance en ligne et en colonne de 9 LEDs sur une carte dont les emplacements sont agencés de manière matricielle. De manière similaire la figure 5B illustre un exemple de répartition à équidistance en ligne et en colonne de 4 LEDs avec un écartement entre les LEDs qui diffère de celui choisi à la figure 5A.

La figure 6A illustre un exemple de répartition à équidistance en diagonale de 18 LEDs sur une carte dont les emplacements sont agencés de manière matricielle. La figure 6B est une variante de la figure 6A selon laquelle les LEDs sont agencées en quatre groupes équidistants les uns des aux autres en ligne et en colonne.

La figure 7A illustre un exemple de répartition à équidistance de 8 LEDs sur une carte dont les emplacements sont agencés en triangles équilatéraux. De manière similaire, la figure 7B illustre un exemple de répartition à équidistance en triangle équilatéral de 3 LEDs avec un écartement entre les LEDs qui diffère de celui choisi à la figure 7A.

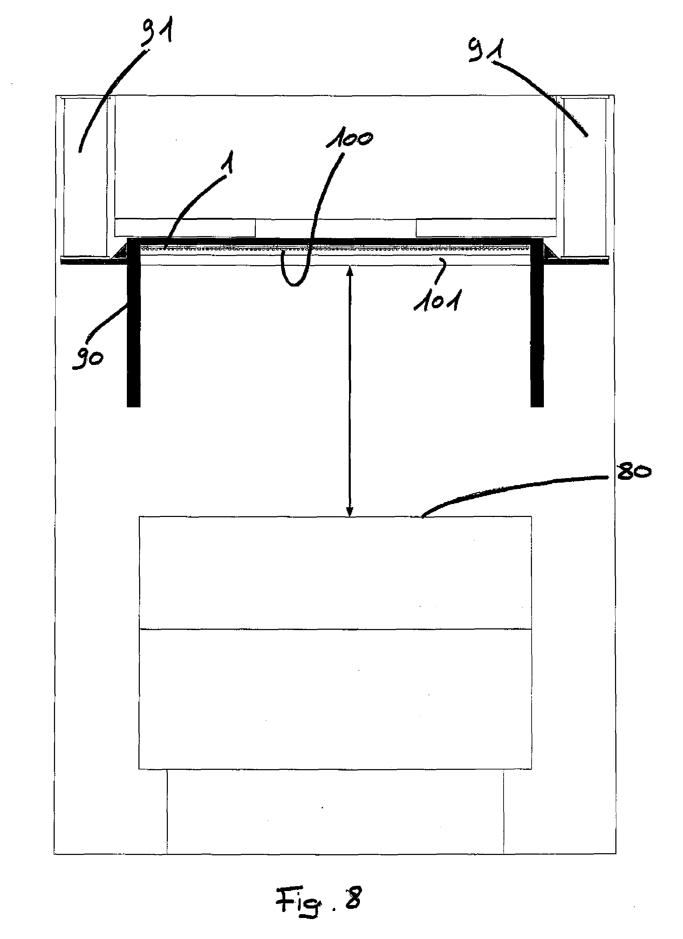

Un exemple de montage équidistant des LEDs de différentes couleurs suivant les proportions relatives obtenues est illustré à la figure 8. Les LEDs de différentes couleurs sont schématisées par des ronds hachurés différemment.

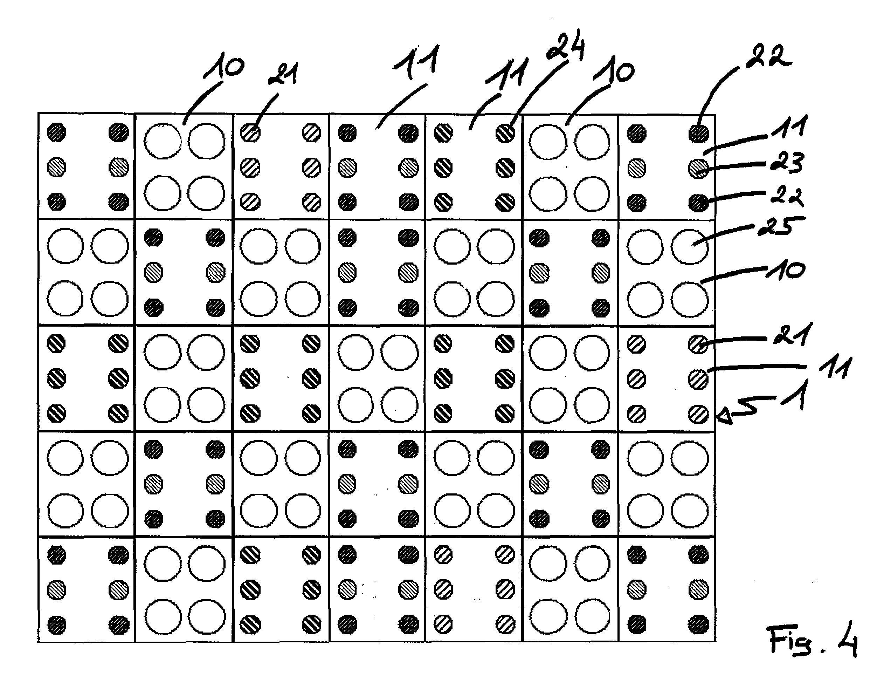

Selon un autre mode de réalisation illustré à la figure 4, on peut prévoir de disposer sur la carte ou chacune des cartes des modules de plusieurs LEDs. Dans l'exemple illustré à la figure 4, il est prévu des modules 10 formés de quatre LEDs pour les LEDs blanches 25 extra-luminescentes et des modules 11 de six LEDs pour les autres LEDs UV 21, rouges "foncé" 22, rouges "clair" 23, infrarouges 24, dites monochromatiques.

Dans l'exemple illustré à la figure 4, les modules 11 de six LEDs présentent trois configurations. Un premier type de module 11 est formé de six LEDs UV, un deuxième type est formé de six LEDs infrarouges, et un troisième type de module 11 comprend deux paires de LEDs rouges "foncé" entre lesquelles sont disposées une paire de LEDs rouges "clair".

Dans cet exemple, les LEDs d'un même module sont alimentées de la même manière. Le pilotage de la carte est ainsi réalisé module par module et/ou par groupes de modules. En variante, on pourrait prévoir un pilotage LED par LED.

### Pilotage du panneau de LEDs

Le panneau comprend des moyens de pilotage conçus pour, d'une part, piloter les LEDs de chaque carte indépendamment des LEDs des autres cartes et, d'autre part, piloter les LEDs (ou modules de LEDs) d'une même carte indépendamment des autres LEDs (ou des modules de LEDs) de ladite carte. Autrement dit, le panneau est pilotable carte par carte et chaque carte est pilotable LED par LED (ou module par module).

A cet effet, le panneau est équipé d'un système électronique et/ou informatique comprenant de manière classique une mémoire et un microprocesseur programmé pour communiquer avec chaque carte selon un adressage donné, et avec chaque LED (ou modules de LEDs) par une sous-adresse correspondante. Les caractéristiques, telles que la couleur, la puissance et l'emplacement des différentes LEDs sur la carte sont mémorisées dans la mémoire des moyens de pilotage. Le pilotage des LEDs s'effectue à l'aide d'instructions programmées dans le microprocesseur. Avantageusement, le système informatique de ladite unité de détermination des LEDs à utiliser pour reproduire un spectre donné, décrite ci-avant, peut être commun à celle des moyens de pilotage.

On peut également prévoir que les LEDs d'une même couleur d'une carte ou les LEDs d'une même couleur de chaque carte puissent être pilotées simultanément.

Préférentiellement, on enregistre des consignes d'alimentation des LEDs d'une carte et lesdites consignes sont répliquées aux autres cartes de manière à obtenir un spectre le plus uniforme possible d'une carte à une autre.

On peut prévoir un scénario de pilotage selon lequel on augmente progressivement l'intensité lumineuse de l'ensemble des LEDs (ou modules de LEDs) pour simuler l'évolution du spectre solaire à l'aurore. De la même manière, on peut prévoir de diminuer progressivement l'intensité d'alimentation des diodes pour simuler l'évolution du spectre solaire au crépuscule. Les différentes séquences de pilotage de LEDs peuvent ainsi être mémorisées pour reproduire les variations d'ensoleillement sur une journée.

Il est également possible d'éteindre ou de faire varier l'intensité d'alimentation d'au moins une partie des LEDs ou d'au moins une partie des groupes de LEDs d'une même couleur pour chaque carte ou pour une partie des cartes, de manière à modifier la qualité du spectre. Un tel pilotage permet par exemple de simuler des trous dans la couche d'ozone affectant le spectre ou encore simuler le passage d'un nuage affectant le niveau d'irradiance dudit spectre.

On peut prévoir au cours du pilotage de l'alimentation des cartes du panneau d'éteindre certaines cartes, par exemple pour simuler une ombre portée (du fait de la présence de feuilles) sur une partie de l'écosystème éclairé.

Comme illustré à la figure 9, ledit panneau est monté sur un châssis 90 mobile à translation selon une direction normale au plan dudit panneau, de manière à permettre un réglage de la distance entre les LEDs dudit panneau 100 et l'écosystème 80 à éclairer. A cet effet, le panneau est monté solidaire d'au moins deux colonnes télescopiques 91 elles-mêmes solidarisées à un plafond. Le déplacement des colonnes télescopiques 91 s'effectue de manière synchronisée.

Grâce à la mobilité de déplacement du panneau par rapport à l'écosystème, on peut également faire varier la puissance reçue par le système en faisant varier la distance entre le panneau de LEDs et l'écosystème, la puissance variant selon l'inverse du carré de la distance.

On peut également prévoir que ledit panneau soit monté mobile selon au moins une plage angulaire donnée autour du ou des éléments à éclairer de manière à permettre une modification de l'inclinaison du panneau, par exemple pour simuler les modifications d'incidence lumineuse au cours des saisons.

Pour la reproduction d'un nouveau spectre de référence à l'aide d'une nouvelle répartition de LEDs sur la carte, on charge ledit spectre de référence dans le modèle mathématique et on relance la génération de spectres de prédiction pour identifier à partir des spectres élémentaires enregistrés affectés de coefficients, le spectre de prédiction le plus proche du nouveau spectre de référence à reproduire. Eventuellement, précédemment à l'opération de génération des spectres de prédiction, on peut avoir enregistré de nouveaux spectres élémentaires pour compléter la base de données de spectres élémentaires accessibles au modèle mathématique.

Ainsi, une base de données de spectres élémentaires suffisamment riche permet de simuler rapidement un spectre de mélange de LEDs proche d'un spectre de référence donné à reproduire. Ledit spectre est alors aisément reproduit et de manière fiable en positionnant les LEDs de différentes couleurs selon les proportions relatives déterminées. Il est alors possible de reconstituer n'importe quel spectre de référence.

Selon un mode de réalisation particulier, on peut prévoir, d'une part, d'agencer des LEDs sur une partie des cartes du panneau selon des proportions relatives de couleurs correspondant à un premier spectre de référence et, d'autre part, d'agencer des LEDs sur une autre partie des cartes du panneau selon des proportions relatives de couleurs correspondant à un deuxième spectre de référence. Il est alors possible de piloter les cartes du panneau de manière à appeler l'un ou l'autre des spectres, ou encore d'activer chacune des cartes afin d'obtenir une combinaison des deux spectres de références.

La présente invention n'est nullement limitée aux modes de réalisation décrits et représentés, mais l'homme du métier saura y apporter toute variante conforme à son esprit.

Lorsque l'on simule les spectres de prédiction pour identifier un spectre de mélange de LEDs s'approchant le mieux du spectre de référence, on peut également imposer une contrainte au modèle mathématique implémenté dans l'unité de détermination des LEDs, permettant de générer le spectre de prédiction, c'est-à-dire imposer un ou plusieurs paramètres de LEDs, tels que le choix d'une puissance donnée pour les LEDs d'une couleur donnée. Le modèle fait alors varier les paramètres des LEDs non imposés pour simuler différents spectres de prédiction et identifier le plus proche du spectre de référence.

On peut prévoir, comme illustré à la figure 9, de disposer une paroi diffusante 101 devant le panneau de LEDs. Dans ce cas, les enregistrements de spectres élémentaires peuvent être réalisés avec ou sans paroi diffusante positionnée entre l'ensemble des LEDs et le spectromètre.

Bien entendu, la description ci-dessus s'applique également dans le cas où le panneau ne comporte qu'une seule carte. Dans ce cas, les LEDs peuvent être pilotées une à une, par groupes et également par couleur.

Chaque carte est également équipée de résistances qui sont montées de manière amovible sur ladite carte de manière à permettre la réalisation de différents circuits de LEDs.

## Revendications

1. Procédé de reproduction d'un spectre lumineux dit de référence (SR), correspondant à une courbe de flux de rayonnement en fonction de la longueur d'onde, tel que le spectre du soleil à un moment donné et à un endroit donné, ledit spectre lumineux étant reproduit à l'aide d'au moins une carte (1) support de diodes électroluminescentes, appelées LEDs (21, 22 23, 24, 25), ledit procédé comportant au moins les étapes suivantes :
a) on allume un nombre donné de LEDs (21) d'une même couleur et on enregistre le spectre (SE1), dit élémentaire, obtenu avec lesdites LEDs (21) allumées,
b) on répète plusieurs fois l'étape a) avec au moins des LEDs (22 ; 23 ; 24; 25) d'une couleur différente à chaque fois,
c) on simule des spectres dits de prédiction, chaque spectre de prédiction étant obtenu en affectant au flux de rayonnement de chaque spectre élémentaire (SE1, SE2, SE3, SE4, SE5) enregistré un coefficient et en sommant lesdits spectres élémentaires affectés de leur coefficient,
d) on identifie parmi les spectres de prédiction simulés, celui (SPP) qui s'approche le plus du spectre de référence (SR) à reproduire,
e) on détermine les proportions relatives de LEDs de chaque couleur à positionner sur ladite au moins une carte à partir au moins du coefficient affecté à, et si nécessaire du nombre de LEDs de, chaque spectre élémentaire du spectre de prédiction identifié comme s'approchant le plus du spectre de référence à reproduire,
f) on dispose sur ladite au moins une carte les LEDs des différentes couleurs selon les proportions relatives déterminées.

2. Procédé selon la revendication 1, **caractérisé en ce que**, la carte utilisée étant une carte présentant des emplacements de réception de LEDs répartis de manière régulière, en ligne et en colonne, ou en triangles équilatéraux, on dispose les LEDs d'une même couleur de telle sorte que lesdites LEDs ou les groupes de LEDs de ladite couleur soient sensiblement équidistants les un(e)s

3. Procédé selon l'une des revendications précédentes, **caractérisé en ce qu'**on monte les LEDs, une à une ou par modules formés de plusieurs LEDs, de manière amovible sur la carte, de préférence par encliquetage.

4. Procédé selon l'une des revendications précédentes, **caractérisé en ce qu'**on intercale sur ladite au moins une carte support les LEDs ou groupes de LEDs d'une même couleur entre des LEDs ou groupes de LEDs d'une ou de plusieurs autres couleurs.

5. Procédé selon l'une des revendications précédentes, **caractérisé en ce qu'**on dispose sur la carte, en plus des LEDs de couleur dont les proportions ont été déterminées à la suite des étapes c) a à f), des LEDs supplémentaires appelées LEDs de réserve.

6. Procédé selon la revendication précédente, **caractérisé en ce que**, pour générer un nouveau spectre à partir des LEDs présentes sur la carte, on simule des spectres de prédiction à partir desdites LEDs et notamment des LEDs de réserve pour identifier la combinaison de LEDs permettant d'obtenir un spectre simulé le plus proche du nouveau spectre souhaité.

7. Procédé selon l'une des revendications précédentes, **caractérisé en ce qu'**on mesure le spectre émis correspondant à la disposition des LEDs sur la carte réalisée à l'étape f), et on modifie la puissance émise par au moins une partie des LEDs, et/ou on pilote au moins une partie des LEDs de réserve lorsqu'elles sont présentes, pour réduire l'écart entre le spectre de référence et le spectre obtenu avec les LEDs disposées sur la carte.

8. Carte (1) de LEDs obtenue selon le procédé de reproduction conforme à l'une des revendications précédentes, **caractérisée en ce que** la carte comporte des emplacements de réception de LEDS qui sont conçus pour un montage amovible des LEDs ou de modules formés de plusieurs LEDs, de préférence par encliquetage, ladite carte étant également, de préférence, équipée de résistances montées de manière amovible sur ladite carte.

9. Carte (1) de LEDs selon la revendication 8, **caractérisée en ce que** ladite carte est équipée de moyens de pilotage de l'alimentation électrique de chaque LED indépendamment des autres LEDs de la carte ou de chaque module de LEDs indépendamment des autres modules de LEDs de la carte.

10. Panneau (100) de LEDs formé d'une pluralité de cartes de LEDs, de préférence conformes à l'une des revendications 8 et 9, dont la répartition des LEDs sur chaque carte est obtenue selon un procédé conforme à l'une des revendications 1 à 7, **caractérisé en ce que** les cartes sont disposées sensiblement côte à côte pour former une surface d'éclairage.

11. Panneau (100) de LEDs selon la revendication 10, **caractérisé en ce que** la répartition des LEDs est sensiblement identique d'une carte à une autre.

12. Panneau (100) de LEDs selon la revendication 10, **caractérisé en ce que** la distribution de LEDs sur une partie des cartes correspond à un spectre de référence donné et la distribution de LEDs sur une autre partie des cartes correspond à un autre spectre de référence donné.

13. Panneau (100) de LEDs selon l'une des revendications 10 à 12, **caractérisé en ce qu'**il comporte des moyens de pilotage conçus pour permettre le pilotage des LEDs d'une carte indépendamment des LEDs des autres cartes et, de préférence, conçus pour permettre le pilotage des LEDs d'une même carte indépendamment des autres LEDs de la carte.

14. Panneau (100) de LEDs selon l'une des revendications 10 à 13, **caractérisé en ce que** ledit panneau est monté mobile à translation selon au moins une direction normale au plan dudit panneau, de manière à permettre un réglage de la distance entre l'ensemble des LEDs dudit panneau (100) et le ou les éléments (80) destinés à être éclairés par ledit panneau, ledit panneau étant également, éventuellement, monté mobile selon au moins une plage angulaire donnée autour du ou des éléments à éclairer de manière à permettre une modification de l'inclinaison du panneau, par exemple pour simuler les modifications d'incidence lumineuse au cours des saisons.

15. Procédé de pilotage des LEDs d'une carte (1) conforme à l'une des revendications 8 et 9 ou d'un panneau (100) de LEDs conforme à l'une des revendications 10 à 14, **caractérisé en ce qu'**on éteint ou on fait varier la puissance émise d'au moins une partie des LEDs de manière à modifier la qualité du spectre.
